# NEW EUROPEAN PATENT SPECIFICATION

(11) **EP 0 390 323 B2**
(45) Date of publication and mention of the opposition decision: **08.08.2012**
(45) Mention of the grant of the patent: 30.12.1998
(21) Application number: 90301817.4
(22) Date of filing: 20.02.1990
(51) Int. Cl.: C12Q 1/68, C07K 14/47, C12N 15/12, C07H 21/04, A61K 48/00

(54) **Detection of loss of the wild-type p53 gene**
Nachweis des Ausfalls des Wildtyps des p53-Gens
Détection de l'écoulement du type sauvage du p53 gène

(30) Priority: 29.03.1989 US 330566
(43) Date of publication of application: 03.10.1990
(73) Proprietor: JOHNS HOPKINS UNIVERSITY, Baltimore, MD 21205-2109 (US)
(72) Inventor: Vogelstein, Bert, Baltimore, MD 21208 (US); Baker, Suzanne J., Baltimore, MD 21218 (US); Fearon, Eric R., Baltimore, MD 21218 (US); Nigro, Janice M., Baltimore, MD 21210 (US)
(74) Representative: Tombling, Adrian George

(56) References cited:
- EP-A1- 0 237 362
- EP-B1- 0 259 031
- WO-A1-88/09387
- VOGELSTEIN B. ET AL.: 'Genetic alterations during colorectal-tumor development' THE NEW ENGLAND JOURNAL OF MEDICINE vol. 319, no. 9, 01 September 2988, pages 525 - 532
- PONDER B.: 'Gene losses in human tumours' NATURE vol. 335, 29 September 2988, pages 400 - 402, XP003010403
- VAN DEN BERG ET AL.: 'Expression of the nuclear oncogene p53 in colon tumours' J OF PATHOLOGY vol. 157, no. 3, April 1989, pages 193 - 199, XP003010404
- 'Genomic p53 gene immortalises' ONCOGENE vol. 2, May 1988, pages 419 - 420, XP003010405
- TAKAHASHI ET AL.: 'p53: A frequent target for genetic abnormalities in lung cancer' SCIENCE vol. 246, 27 October 1989, pages 491 - 494, XP003010406
- BAKER S.J. ET AL.: 'Chromosome 17 deletions and p53 gene mutations in colorectal carcinomas' SCIENCE vol. 244, 14 April 1989, pages 217 - 221, XP003010407
- COTTON R.G.H. ET AL.: 'Reactivity of cytosine and thymine in single-base-pair mismatches with hydroxylamine and osmium tetroxide and its application to the study of mutations' PNAS vol. 85, 1988, pages 4397 - 4401, XP002086182
- ELIYAHU D. ET AL.: 'Meth a fibrosarcoma cells express two transforming mutant p53 species' ONCOGENE vol. 3, September 1988, pages 313 - 321, XP009003748
- The EMBO Journal, vol. 4 (1985) pp.1251-1255
- Molecular and Cellular Biology, vol. 6 (1986) pp.1379-1385
- Molecular and Cellular Biology, vol. 6 (1986) pp.4650-4656
- Molecular and Cellular Biology, vol. 7 (1987) pp.961-963
- Gene, vol. 70 (1988) pp.245-252

## Description

The invention relates to the area of cancer diagnostics. More particularly, the invention relates to detection of the loss and or alteration of wild-type p53 genes from tumour tissues.

Recent studies have elucidated several genetic alterations that occur during the development of colorectal tumours. the most common of which are deletions of the short arm of chromosome 17 (17p). While some genetic alterations such as RAS gene mutations appear to occur relatively early during colorectal tumour development, chromosome 17p deletions are often late events associated with the transition from the benign (adenomatous) to the malignant (carcinomatous) state. See Vogelstein et al., New England Journal of Medicine, Vol. 319, p525, 1988.

Because carcinomas are often lethal, while the precursor adenomas are uniformly curable, the delineation of the molecular events mediating this transition are of considerable importance. The occurrence of allelic deletions of chromosome 17p in a wide variety of cancers besides those of the colon, including those of the breast and lung, further emphasizes the importance of genes residing on chromosome 17p in the neoplastic process. Because allelic deletions have been reported to encompass a large area of chromosome 17p, there is a need in the art for defining the particular genetic region which is responsible for the neoplastic progression.

Oncogene, vol 2 (1988) pp. 419-420 and Oncogene, vol 3 (1988) pp. 313-321 disclose the wild-type p53 gene as being a protooncogene, the mutant gene thereof being strongly oncogenic.

The invention that is the subject of this application is defined in the attached claims.

The present invention provides the art with the information that the p53 gene is, in fact, the target of both deletional and point mutational alterations on chromosome 17p which are associated with the process of tumorigenesis. This information allows highly specific assays to be done to assess the neoplastic status of a particular tumor tissue.

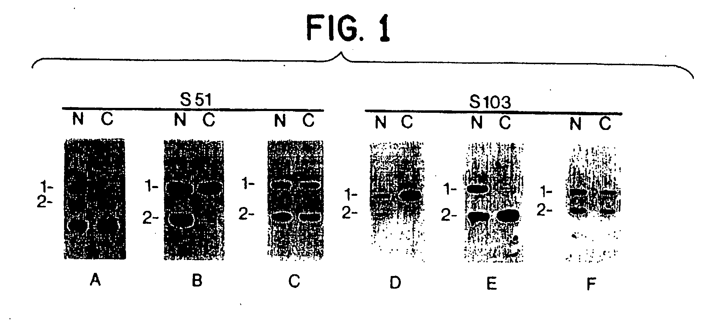

Figure 1 demonstrates the analysis of allelic losses on chromosome 17p in the human tissue of two patients, S51 and Si03.

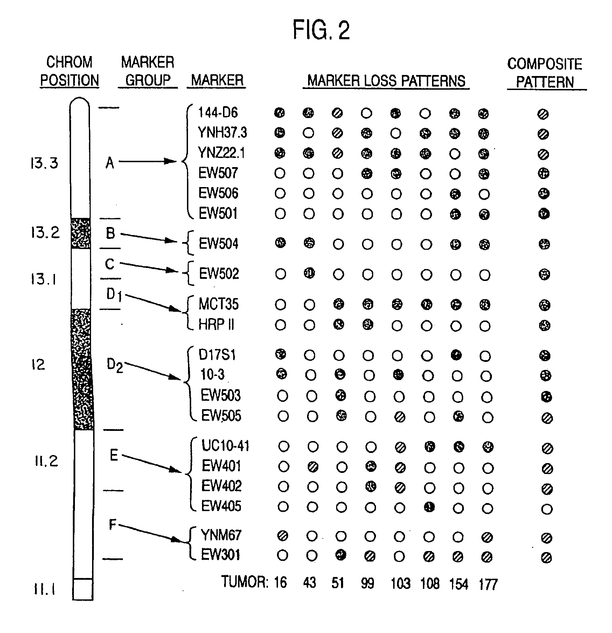

Figure 2 shows a map of the common region of deletions on chromosome 17p in colorectal tumors. The chromosomal positions of 20 restriction fragment length polymorphism (RFLP) markers from chromosome 17p are indicated. The markers were previously mapped to seven sub-chromosomal regions (indicated A to F). Hybridization results for eight tumors are shown on the right, with patient identification numbers indicated at the bottom. A filled circle indicates loss of one parental allele in the tumor, a cross-hatched circle indicates retention of both parental alleles; an open circle indicates that the marker was not informative, i.e. the patient's normal tissue was not heterozygous for the marker. The premise of the composite pattern is that there is a single target gene on 17p. Therefore, markers for which heterozygosity was retained in any of the eight tumors (i.e., cross-hatched circles) would be outside the target locus.

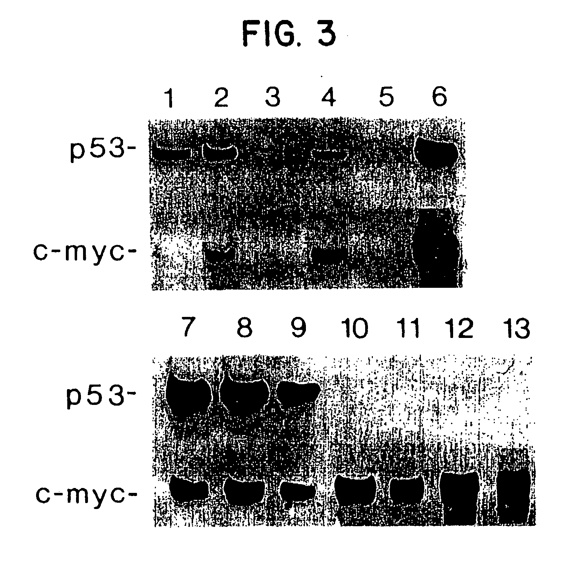

Figure 3 shows a Northern blot analysis of p53 mRNA in colorectal tumors. The RNA in Lanes 1-6 and 12 was prepared from human tissues (normal colonic mucosa (N) or carcinoma biopsies (C)). The RNA in lanes 7-11 and 13 was prepared from colorectal carcinoma cell lines.

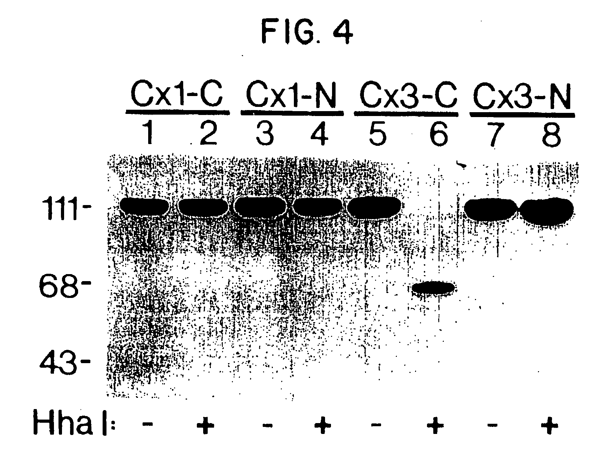

Figure 4 shows analysis of the products of polymerase chain amplification of a 111 bp fragment surrounding the p53 gene codon 143. Lanes 1.2: colorectal tumor xenograft Cx1; lanes 3,4: normal fibroblasts from the patient providing Cx1; lanes 5,6: colorectal tumor xenograft Cx3; lanes 7,8: normal fibroblasts from the patient providing Cx3.

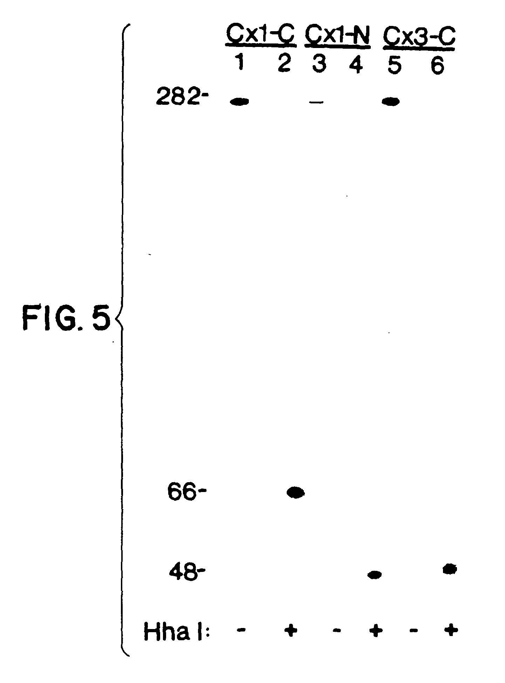

Figure 5 shows polymerase chain reaction analysis of p53 codon 175. Lanes 1,2: colorectal tumor xenograft Cx1; lanes 3,4: normal fibroblasts from the patient providing Cx1; lanes 5,6: colorectal tumor xenograft Cx3. Samples in even numbered lanes only were digested with Hha I.

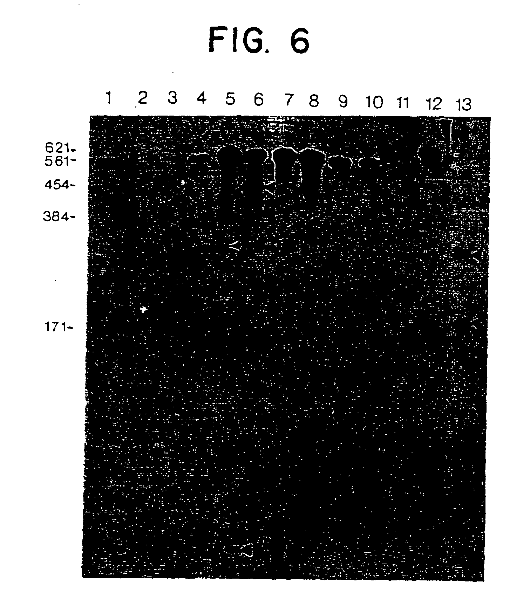

Figure 6 depicts RNase protection analysis of p53 mRNA. Cellular RNA was hybridized with radiolabeled anti-sense p53 RNA probe, and the hybrids digested with RNase A. The RNA was derived from: lane 1: S115, carcinoma biopsy; lane 2: SW1417; lane 3: SW948; lane 4: RKO; lane 5: SW480; lane 6: RCA; lane 7: GEO; lane 8 FET lane 9: xenograft Cx3; lane 10: normal colonic mucosa; lane 11: yeast tRNA; lane 12: probe alone (not RNase A digested); lane 13: SW1417 (long exposure). The fragments marked with arrowheads in lanes 5.6, and 13 were not present in the other samples.

It is a discovery of the present invention that mutational events associated with tumorigenesis occur in the p53 gene on chromosome 17p. Although it was previously known that deletion of alleles on chromosome 17p were common in certain types of cancers, it was not known that the deletions shared a common region which includes the p53 gene. Further it was not known that a second mutational event on the sister chromosome of that carrying the deletions was also affected by mutation in the p53 gene. The mutation of the sister chromosome does not involve gross rearrangements such as deletions, insertions or inversions, but rather point mutations located in a variety of positions throughout the p53 gene. Although the inventor does not wish to be bound by the following theory, it is proposed as a possible mechanism which explains the observed result. It is believed that the point mutation occurs first and the deletion event occurs second, as the latter event is correlated with the change of a tumor from an adenomatous to a carcinomatous state.

According to the diagnostic method of the present invention, loss of the wild-type p53 gene is detected. The loss may be due to either deletional and/or point mutational events. If only a single p53 allele is mutated, an early neoplastic state is indicated. However, if both alleles are mutated then a late neoplastic state is indicated. The p53 allele which is not deleted (i.e., that on the sister chromosome to the chromosome carrying the deletion) can be screened for point mutations, such as missense, and frameshift mutations. Both of these types of mutations would lead to non-functional p53 gene products. In addition, point mutational events may occur in regulatory regions, such as in the promoter of the p53 gene, leading to loss or diminution of expression of the p53 mRNA.

In order to detect the loss of the p53 wild-type gene in a tissue, it is helpful to isolate the tissue free from surrounding normal tissues. Means for enriching a tissue preparation for tumor cells are known in the art. For example, the tissue may be isolated from paraffin or cryostat sections. Cancer cells may also be separated from normal cells by flow cytometry. These as well as other techniques for separating tumor from normal cells are well known in the art. If the tumor tissue is highly contaminated with normal cells, detection of mutations is more difficult.

Detection of point mutations may be accomplished by molecular cloning of the p53 allele (or alleles) present in the tumor tissue and sequencing that allele(s) using techniques well known in the art. Alternatively, the polymerase chain reaction can be used to amplify p53 gene sequences directly from a genomic DNA preparation from the tumor tissue. The DNA sequence of the amplified sequences can then be determined. The polymerase chain reaction itself is well known in the art. See e.g., Saiki et al., Science, Vol. 239, p. 487, 1988: U.S. 4,683,203; and U.S. 4,683,195. Specific primers which can be used in order to amplify the p53 gene will be discussed in more detail below.

Specific deletions of p53 genes can also be detected. For example, restriction fragment length polymorphism (RFLP) probes for the p53 gene or surrounding marker genes can be used to score loss of a p53 allele. Other techniques for detecting deletions, as are known in the art can be used.

Loss of wild-type p53 genes may also be detected on the basis of the loss of a wild-type expression product of the p53 gene. Such expression products include both the mRNA as well as the p53 protein product itself. Point mutations may be detected by sequencing the mRNA directly or via molecular cloning of cDNA made from the mRNA. The sequence of the cloned cDNA can be determined using DNA sequencing techniques which are well known in the art. The cDNA can also be sequenced via the polymerase chain reaction (PCR) which will be discussed in more detail below

Alternatively, mismatch detection can be used to detect point mutations in the p53 gene or its mRNA product. While these techniques are less sensitive than sequencing, they are simpler to perform on a large number of tumors. An example of a mismatch cleavage technique is the RNase protection method which is described in detail in Winter et al., Proc. Natl. Acad. Sci. USA, Vol. 82, p. 7575. 1985 and Meyers et al., Science. Vol. 230, p. 1242. 1985. In the practice of the present invention the method involves the use of a labeled riboprobe which is complementary to the human wild-type p53 gene. The riboprobe and either mRNA or DNA isolated from the tumor tissue are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the p53 mRNA or DNA. The riboprobe need not be the full length of the p53 mRNA or gene but can be a segment of either. If the riboprobe comprises only a segment of the p53 mRNA or gene it will be desirable to use a number of these probes to screen the whole mRNA sequence for mismatches.

In similar fashion, DNA probes can be used to detect mismatches, through enzymatic or chemical cleavage. See, e.g.. Cotton et al.. Proc. Natl. Acad. Sci. USA, vol. 85, 4397, 1988; and Shenk et al.. Proc. Natl. Acad. Sci. USA, vol. 72. p. 989. 1975. Alternatively, mismatches can be detected by shifts in the electrophoretic mobility of mismatched duplexes relative to matched duplexes. See, e.g., Cariello, Human Genetics, vol. 42, p. 726, 1988. With either ribo-probes or DNA probes, the cellular mRNA or DNA which might contain a mutation can be amplified using PCR (see below) before hybridization.

DNA sequences of the p53 gene from the tumor tissue which have been amplified by use of polymerase chain reaction may also be screened using allele-specific probes. These probes are nucleic acid oligomers, each of which contains a region of the p53 gene sequence harboring a known mutation. For example, one oligomer may be about 30 nucleotides in length, corresponding to a portion of the p53 gene sequence. At the position coding for the 175th codon of p53 gene the oligomer encodes an alanine, rather than the witd-type codon valine. By use of a battery of such allele-specific probes, the PCR amplification products can be screened to identify the presence of a previously identified mutation in the p53 gene. Hybridization of allele-specific probes with amplified p53 sequences can be performed, for example, on a nylon filter. Hybridization to a particular probe indicates the presence of the same mutation in the tumor tissue as in the allele-specitic probe.

Loss of wild-type p53 genes can also be detected by screening for loss of wild-type p53 protein function. Although all of the functions which the p53 protein undoubtedly possesses have yet to be elucidated, at least two specific functions are known. Protein p53 binds to the SV40 large T antigen as well as to the adonovirus E1B antigen. Loss of the ability of the p53 protein to bind to either or both of these antigens indicates a mutational alteration in the protein which reflect a mutational alteration of the gene itself. Alternatively, a panel of monoclonal antibodies could be used in which each of the epitopes involved in p53 functions are represented by a monoclonal antibody Loss or perturbation of binding of a monoclonal antibody in the panel would indicate mutational alternation of the p53 protein and thus of the p53 gene itself. Any means for detecting an altered p53 protein can be used to detect loss of wild-type p53 genes.

Mutant p53 genes or gene products can also be detected in body samples, such as, stool, or other body fluids, such as urine and sputum. The same techniques discussed above for detection of mutant p53 genes or gene products in tissues can be applied to other body samples. By screening such body samples, a simple early diagnosis can be achieved for many types of cancers. In addition, the progress of chemotherapy or radiotherapy can be monitored more easily by testing such body samples for mutant p53 genes or gene products.

The method of the present invention for diagnosis of neoplastic tissue is applicable across a broad range of tumors. These include lung, breast, brain, colorectal, bladder, prostate, liver as well as stomach tumors. In addition the method may be used in leukemias and osteosarcomas. It thus appears that the p53 gene has a role in the development of a broad range of tumors. The methods of diagnosis of the present invention are applicable to any tumor in which p53 has a role in tumorigenesis. The diagnostic method of the present invention is useful for clinicians so that they can decide upon an appropriate course of treatment. For example, a tumor displaying loss of both p53 alleles suggests a more aggressive therapeutic regimen than a tumor displaying loss of only one p53 allele.

The nucleic acid probes are useful in the RNase protection method for detecting point mutations already discussed above. They may also be used to detect mismatches with the p53 gene or mRNA using other techniques. Mismatches can be detected using other enzymes (e.g., S1 nuclease), chemicals (e.g.. hydroxylamine or osmium tetroxide and piperidine), or changes in electrophoretic mobility of mismatched hybrids as compared to totally matched hybrids. These techniques are known in the art. See. Cotton, supra, Shenk, supra. Myers, supra, Winter, supra, and Novack et al.. Proc. Natl. Acad. Sci. USA. vol. 83. p. 586, 1986. If a riboprobe is used to detect mismatches with mRNA, it is complementary to the mRNA of the human wild-type p53 gene. The riboprobe thus is an anti-sense probe in that it does not code for the p53 protein because it is of the opposite polarity to the sense strand. The riboprobe generally will be radioactively labeled which can be accomplished by any means known in the art. If the riboprobe is used to detect mismatches with DNA it can be of either polarity, sense or anti-sense. Similarly, DNA probes also may be used to detect mismatches. Probes may also be complementary to mutant alleles of p53 gene. These are useful to detect similar mutations in other patients on the basis of hybridization rather than mismatches. These are discussed above and referred to as allele-specific probes.

The present invention also includes the in vitro use of a human wild-iype p53 gene for the restoration of wild-type p53 function to a cell which carries mutant p53 alleles. The wild-type p53 gene or a part of the gene may be introduced into the cell in a vector such that the gene remains extrachromosomal. In such a situation the gene will be expressed by the cell from the extrachromosomal location. If the mutant p53 genes present in the cell are expressed, then the wild-type p53 gene or gene portion should be expressed to a higher level than that of the mutant gene. This is because the mutant forms of the protein are thought to oligomerize with wild-type forms of the protein. (Eliyahu et al., Oncogene. Vol. 3, p. 313, 1988.) If a gene portion is introduced and expressed in a cell carrying a mutant p53 allele, the gene portion should encode a part of the p53 protein which is required for non-neoplastic growth of the cell. More preferred is the situation where the wild-type p53 gene or a part of it is introduced into the mutant cell in such a way that it recombines witch the endogenous mutant p53 gene present in the cell. Such recombination would require a double recombination event which would result in the correction of the p53 gene mutation. Vectors for introduction of genes both for recombination and for extrachromosomal maintenance are known in the art and any suitable vector may be used.

Polypeptides or other molecules which have p53 activity may be supplied to cells which carry mutant p53 alleles. The active molecules can be introduced into the cells by microinjection or by liposomes, for example. Alternatively some such active molecules may be taken up by the cells, actively or by diffusion. Supply of such active molecules will effect an earlier neoplastic state.

Predisposition to cancers can be ascertained by testing normal tissues of humans. For example, a person who has inherited a germline p53 mutation would be prone to develop cancers. This can be determined by testing DNA from any tissue of the person's body. Most simply, blood can be drawn and DNA extracted from the cells of the blood. Loss of a wild-type p53 allele, either by point mutation or by deletion, can be detected by any of the means discussed above. DNA can also be extracted and tested from fetal tissues for this purpose.

The following are provided for exemplification purposes only and are not intended to limit the scope of the invention which has been described in broad terms above.

### Example 1:

This example demonstrates that the deletions found on chromosome 17p in human colorectal carcinomas share a common region between bands 17p12 and 17p13.3.

Twenty DNA probes detecting restrictions fragment length polymorphisms (RFLPs) on chromosome 17p were used to examine the patterns of allelic losses in colorectal tumors. These probes have been mapped to seven discrete regions of 17p on the basis of their hybridization to human-rodent somatic cell hybrids containing parts of chromosome 17p (P. van Tuinen. D.C. Rich. K.M. Summers, D.H. Ledbetter, Genomics 1. 374 (1987); P. van Tuinen et al., Am. J. Hum. Gen. 43, 587 (1988); P.R. Fain et al.. Genomics 1, 340 (1987); unpublished data of D.H. Ledbetter and D.F. Barker).

DNA was obtained from 58 carcinoma specimens and compared to DNA from adjacent normal colonic mucosa. Allelic losses were scored it either of the two alleles present in the normal cells was absent in the DNA from the tumor cells. Allelic deletions can be difficult to detect in DNA prepared from whole tumors because most solid tumors contain a significant number of non-neoplastic stromal and inflammatory cells. For this reason, regions of tumors containing a high proportion of neoplastic cells were identified histopathologically and isolated, and DNA was prepared from cryostat sections of these regions as described previously (S. Goelz. S.R. Hamilton. B. Vogelstein, Biochem. Biophys. Res. Commun. 130, 118 (1985): E.R. Fearon, A. Feinberg, S.R. Hamilton, B. Vogelstein, Nature 318, 377 (1985). Grossly normal colonic mucosa adjacent to the tumors was obtained from each patient and used to prepare control DNA.

The two parental alleles could be distinguished in the normal mucosa of each patient with at least 5 of the 20 RFLP markers (the "informative" markers for each case). Seventy-seven percent of the tumors exhibited allelic losses of at least 3 markers. Studies of 8 tumors which retained heterozygosity for some but not all markers on chromosome 17p enabled the definition of a common region of deletion.

Figure 1 shows a sample of the data collected from two patents DNA from normal (N) and carcinoma (C) tissue of patients S51 and S103was digested with restriction endonucleases and the fragments separated by electrophoresis. After transfer to nylon filters, the DNA was hybridized to radiolabeled probes. Techniques used for DNA purification restriction endonuclease digestion, electrophoresis, transfer and hybridization were performed as described (B. Vogelstein et al., N. Engl. J. of Med. 319. 525 (1988); Goelz. supra; Fearon, supra.) Taq I digestion was used for panels A. B. C, and F, BamHI for panel D and MspI for panel E. Autoradiographs of the washed filters are shown. The alleles designated "1" and "2" refer to the larger and smaller polymorphic alleles, respectively, present in the normal DNA samples. The probes used were: A: MCT35.2; B: EW301; C: YNH37.3; D: YNZ22.1; E: MCT35.1; F: EW505. Deletions of allele 1 can be seen in panels A and E; deletions of allele 2 in panels B and D.

The tumor from patient S51 had retained both parental alleles of three markers from the distal region of 17p, but had lost one of all more proximal markers that were formative (Fig. 1, A-C). This implied that the target of the allelic loss in this tumor was proximal to the three retained markers. Analysis of the pattern of marker loss is shown in Figure 2. The tumor from patient S103 had retained both parental alleles at all informative loci proximal to EW505, but had allelic deletions of several more distal markers (Fig. 1, D-F). The combined data depicted in Figure 2 indicated that the smallest common region of deletion extended between markers within band 17p12 to those within band 17p13.3. This localization is based on the assumption that the same 17p locus was the target of deletion in all of the tumors.

### Example 2:

This example demonstrates that the non-deleted p53 alleles in colorectal carcinomas carrying a p53 deletion are not rearranged.

First, p53 cDNA probes detecting exons spread over 20,000 base pairs (including all protein encoding exons) R. Zakut-Houri, B. Bienz-Tadmor, D. Givol. M. Oren. EMBO J. 4, 1251 (1985); N. Harris E. Brill, O. Shahat, M. Prokocimer. T.E. Admas, Mol. Cell. Biol., 6, 4650 (1986); G. Matlashewski et al., Molec. Cell. Biol. 7. 961 (1987); V.L. Buchman et al., Gene 70, 245 (1988)] were used to examine the DNA of 82 colorectal carcinomas (50 primary specimens and 32 cell lines) in Southern blotting experiments.

No rearrangements of the p53 gene were observed with EcoRI or BamH I digests, nor were deletions of both alleles seen. Because p53 expression might be affected by gross genetic alterations further removed from p53 coding sequences, pulsed-field gel electrophoresis was used to examine large restriction fragments encompassing the p53 gene. The restriction endonucleases EcoR V, PaeR7 I, Not I, and Sal I generated p53 gene-containing fragments of 45-350kb from the DNA of normal cells. No alterations were detected in the DNA from any of 21 colorectal tumor cell lines examined with each of these four enzymes.

### Example 3:

This example demonstrates that the non-deleted p53 alleles in colorectal carcinomas carrying a p53 deletion express mRNA of the normal size and in most cases normal amount.

Northern blot experiments were performed on RNA from 22 colorectal tumors (6 primary tumors and 16 cell lines). Because p53 expression has been correlated with cellular growth and/or transformation other genes whose expression is similarly regulated were used as controls (c-myc, histone H3, and phosphoghycerate kinase).

RNA was purified from grossly normal colonic mucosa, primary carcinoma specimens or tumor cell lines, and separated by electrophoresis. Cell lines were generously provided by D. and M. Brattain or obtained from the American Type Culture Collection. Rockville, Maryland. Total cellular RNA was isolated by the acid-guanidium extraction method (P. Chomczynski, N. Sacchi, Anal. Biochem. 162, 156 (1987)). Five micrograms were separated by electrophoresis through a 1.5% 2(N-morpholino) ethane sulfonic acid-formaldehyde agarose gel and electrophoretically transferred to nylon filters. The RNA was transferred to nylon filters and hybridized with a radiolabeled p53 gene probe. Labelling of the probes, hybridization, washing and autoradiography were performed as described. (Fearon et al., Science, Vol. 238, p. 193, 1987; Vogelstein et al., N. Engl. J. of Med., Vol. 319, p. 525, 1988; and Goelz, supra; and Fearon. Nature, supra). Autoradiographs were exposed for 18-24 hours.

The p53 probe was a 1.8 kb Xbal fragment of a p53 cDNA clone generously provided by D. Givol (EMBO J., vol. 4, p. 1251 (1985)). The c-myc probe was a 1.6 kb genomic Sstl fragment containing exon 2 of c-myc (K. Alitalo et al.. Proc. Nat'l. Acad. Sci. USA 80, 1707 (1983)). The signals were removed from the filter, and the blot was re-hybridized with a c-myc gene probe. Autoradiographs of the hybridized filters are shown in Figure 3. The size of the p53 mRNA detected was 2.8 kb. and the size of the c-myc mRNA was 2.5 kb.

The RNA in lanes 1-6 and lane 12 was prepared from human tissues (normal colonic mucosa (N) or carcinoma biopsies (C)). The RNA in lanes 7-11 and 13 was prepared from colorectal carcinoma cell lines. Lanes 1, 2: Patient S345, N and C, respectively. Lanes 3, 4: Patient S353. N and C. respectively. Lanes 5, 6, Patient S369, N and C, respectively. Lane 7: SW837, Lane 8: SW480, Lane 9: LoVo, Lane 10: SW948, Lane 11: SW1417, Lane 12: Patient S115, C, Lane 13: RKO.

The size of p53 mRNA was normal (2.8 kb) in all 22 tumors. Moreover, the relative abundance of p53 gene mRNA was usually at least as great in colorectal tumor cells as in normal colonic mucosa confirming the results of Calabretta et al. (Career Research. Vol. 46, p. 738 (1986)). However, in four tumors, (lanes 10-13) relatively little expression of p53 mRNA was observed compared to that in the other tumors. This low level of expression of p53 was specific in that c-myc, histone H3, and phosphoglycerate kinase mRNAs were expressed in these four tumours at levels similar to those seen in other colorectal tumours and at least as high as in non-neoplastic colonic mucosa.

### Example 4:

This example demonstrates that the non-deleted p53 allele in a primary tumour carries a point mutation at codon 143. '

A tumour was chosen which had an allelic deletion of chromosome 17p yet expressed significant quantities of p53 mRNA. A cDNA clone originating from the remaining p53 allele was isolated and sequenced to determine whether the gene product was abnormal.

For practical reasons, a nude mouse xenograft (Cx3) of a Primary tumour was selected for this test. Primary tumours contain non-neoplastic cells which could contribute p53 mRNA, while in xenografts the non-neoplastic cells (derived from the mouse) could not be the source of a human p53 cDNA clone. Cx3, like over 75% colorectal carcinomas, had allelic deletions of several RFLP (restriction fragment length polymorphism) markers on chromosome 17 and expressed significant amounts of p53 mRNA.

A nearly full-length p53 cDNA was cloned from Cx3 mRNA using standard techniques. Double stranded cDNA was synthesized as described by U. Gubler and B. J. Hoffman, Gene 25 263 (1983) and cloned into the lambda g110 vector. The cDNA insert was subcloned into Bluescript KS (trademark) (Stratagene Cloning Systems, LaJolla CA) and nested deletions were made with exonuclease III (S. Henekoff, Gene 28, 351 [1984]). Sequences were obtained from double-stranded templates using modified T7 polymerase as described by S. Tabor and C. C. Richardson, Proc. Natal. Acad. Sci. USA 84, 4767 (1987) and R. Kraft, J. Tardiff, K. S. Krauter and L.A Leinwand, Biotechniques 6, 544 (1988).

The clone extended 2567 nucleotides from position -198 relative to the translation initiation site to the polyadenosine tail. The clone was sequenced by the dideoxy chain-termination method and one nucleotide difference was identified in comparison with published p53 cDNA sequences (See, Zakut-Houri, supra; Harris. supra; Matlashewski; supra; and Buchman, supra). A transition from T to C had occurred within codon 143 (GTG to GCG), resulting in a change of the encoded amino acid from valine to alanine.

To ensure that the sequence change was not an artifact of cDNA cloning, the polymerase chain reaction [PCR, (Saiki, et al., Science, Vol. 239, p. 487, 1988)] was used to amplify a 111 base pair (bp) sequence surrounding the presumptive mutation from genomic DNA of Cx3.

DNA was incubated in the presence of Taq polymearse with primer oligomers complementary to sequences 68 base pairs upstream and 43 base pairs downstream of codon 143. The upstream primer used was 5'-TTCCTCTTC-CTGCAGTACTCC-3'; all but 6 nucleotides of this primer were derived from the p53 intron 4 sequence determined by Buchman et al., supra. The downstream primer was 5'-GACGCGGGTGCCGGGCGG-3'. After 35 cycles of denaturation (one minute, 93°), annealing (2 minutes, 55°) and elongation (2 minutes, 70°) amplified DNA fragments of 111 bp were generated. Following electrophoresis, the 111 bp amplified fragments were eluted from a polyacrylamide gel and purified by extraction with phenol and chloroform.

Analysis of the PCR product was facilitated by the observation that the presumptive mutation created a new Hha I site (GCGC at nt 427-430). An aliquot of each of the purified DNA fragments was digested with Hha I, separated by electrophoresis on a non-denaturing polyracrylamide gel, and electrophoretically transferred to nylon filters. The fragments were hybridized with a radioactive p53 probe generated from a 1.8 kb Xba I fragment of a p53 cDNA clone provided by D. Givol (Zakot-Houri, supra).

The 111 bp PCR product from tumor Cx3 was cleaved with Hha I to produce the expected 68 and 43 subfragments (Figure 4, lanes 5 and 6). The 111 bp PCR product from the DNA of normal cells of the patient providing Cx3 was not cleaved with Hha I (lanes 7 and 8), nor were the PCR products of 37 other DNA samples prepared from the normal tissues, primary colorectal tumors, or xenografts of other patients (examples in Fig. 4, lanes 1-4). Therefore, the valine to alanine substitution present in this tumor was the result of a specific point mutation not present in the germline of the patient.

A small amount of a contaminating 73 base pair PCR product was present in most of the eluates; the contaminant was not cleaved by Hha I, however, so that it did not interfere with the analysis.

### Example 5:

This example demonstrates that a second tumor from a different patient carried a point mutation at codon 175 of the p53 gene.

Colorectal carcinoma xenograft Cx1, like Cx3, had alleic deletions of several markers on chromosome 17p and expressed considerable amounts of normal size p53 mRNA. First strand cDNA was generated from Cx1 RNA using random hexamers in the presence of reverse transcriptase (E. Noonan and I.B. Roninson, Nucleic Acids Research 16, 10366 [1988]). This cDNA was used in five separate PCR reactions to generate fragments corresponding to nucleotides -59 to 246 (primer pair 1), 189 to 508 (primer pair 2). 443 to 740 (primer pair 3), 679 to 979 (primer pair 4), and 925 to 1248 (primer pair 5). These fragments contained all coding sequences of the p53 gene. Primer pair 1: 5'-GGAATTCCACGACGGTGACACG-3' and 5'-GGAATTCGGTGTAGGAGCTGCTGG-3; pair 2: 5'-GGAATTCCCA-GAATGCCAGAGGC-3' and 5'-GGAATTCATGTGCTGTGACTGCTTG-3'; pair 3: 5'-GGAATTCCACACCCCCGCCCG-3' and 5'-GGAATTCATGCCGCCCATGCAG-3'; pair 4: 5'-GGAATTCTGACTGTACCACCATCC-3' and 5'-GGAATTCTC-CATCCAGTGGTTTC-3'; pair 5: 5'-GGAATTCCCAACAACACCAGCTCC-3' and 5'-GGAATTCAAAATGGCAG-GGGAGGG-3'. All primers had extraneous nucleotides comprising EcoR I cleavage sites at their 5' ends to facilitate cloning. The PCR products were cloned in the EcoR I site of Bluescript SK and sequenced as described in Example 4. Only 1 base pair change was identified (transition from CGC to CAC) and this change at codon 175 was found in two independent clones.

To ensure that the sequence change represented a mutation rather than a sequence polymorphism, PCR was used to amplify a fragment containing codon 175 from the genomic DNA of tumor Cx1 and normal cells. PCR was used to amplify a 319 bp fragment containing intron 5 and surrounding exon sequences. The upstream primer was the same as used for primer pair 3 and the downstream primer was 5'-CGGAATTCAGGCGGCTCATAGGGC-3'; PCR was performed as decribed in Example 4. Following electrophoresis through a 2% agarose gel, the 319 bp fragment was purified by binding to glass beads (Vogelstein et al., Proc. Nat'I. Acad. Sci. USA, Vol. 76, p. 615 (1979)). The DNA fragments were cleaved with Sty I at nt 477 and end-labeled by fill-in with the Klenow fragment of DNA Polymerase I and ³²P-dCTP. Following electrophoresis of the reaction mixture through a non-denaturing polyacrylamide gel, the 282 bp Sty I fragment (nt 477-758), labeled at the proximal end and containing codon 175, was eluted and purified by extraction with phenol and cloroform. A portion of the eluted DNA was cleaved with Hha I and the fragments separated by electrophoresis on a 6% sequencing gel. The presumptive mutation abolished the Hha I site normally present at codon 175 (GCGC at nt 522 to 525). Thus, Hha I cleavage of the PCR products from DNA of the normal cells of the patient providing Cx1 (Figure 5, lanes 3 and 4) or from the tumor of another patient (lanes 5 and 6) produced only the 48 bp product expected if codon 175 was wild-type. In contrast, the PCR product from tumor Cx1 was not cleaved at nt 524 (corresponding to codon 175) and exhibited only a larger 66 bp fragment resulting from cleavage at a normal downstream Hha I site at nt 542. Analysis of the PCR product from paraffin embedded samples of the primary tumor and liver metastasis also exhibited the diagnostic 66 bp Hha I fragment indicating the presence of a mutation.

### Example 6:

This example shows that five out of twenty-one carcinomas tested with the RNase protection method produced mRNA molecules with detectable sequence mismatches to the wild-type p53 RNA sequence.

Hybrids between a p53 anti-sense RNA probe and p53 mRNA should be cleaved by RNase A only at sequence mismatches. Although this method is not as definitive or as sensitive as sequencing, it allows rapid screening of a larger number of tumors. Twenty-one colorectal carcinomas (6 primary tumors and 15 cell lines) were examined with probes that included most of the p53 coding region.

Ten ug of cellular RNA was hybridized with radiolabeled anti-sense p53 RNA probe, and the hybrids digested with RNase A. A ³²P-labelled RNA probe was generated in vitro from a p53 cDNA subclone in Bluescript (Stratagene Cloning Systems, La Jolla, CA). The probe included 561 nt of p53 mRNA coding sequence (nt 473-1034 relative to the translation start site) plus 60 nt derived from the vector.

The protected fragments were separated by electrophoresis through denaturing polyacrylamide gels; autoradiographs of the gels are presented in Figure 6. The RNA was derived from: lane 1: S115. carcinoma biopsy; lane 2: SW1417; lane 3: SW948; lane 4: RKO; lane 5: SW480; lane 6: RCA; lane 7: GEO; lane 8: FET, lane 9: xenograft Cx3; lane 10: normal colonic mucosa; lane 11: yeast tRNA: lane 12: probe alone (not digested with RNase A); lane 13: SW1417 (long exposure). The fragments marked with arrowheads in lanes 5, 6 and 13 were not present in the other samples. The autoradiographic exposure time for lane 13 was 72 hours to allow adequate visualization of the new fragments; for all other lanes the exposure time was 10 hours.

The RNA from 5 carcinomas protected fragments of a different size than those seen with RNA from normal cells. In two cases, the new fragments were the major fragments detected (Fig. 6, lanes 6 and 13, arrowheads). In other cases, the new fragments were of minor intensity compared to the fully protected fragment (for example, SW480 in lane 5). Such partial cleavages are not unexpected: the mutations in Cx3 and Cx1 were not detected by the RNase protection method (data not shown) and it is known that the majority of RNA sequence mismatches are partially or totally resistant to RNaseA.

Using similar techniques, five additional colorectal cancers, two breast tumors and one lung tumor have been examined for p53 gene mutations. In all cases, point mutations of the p53 gene were observed.

## Claims

1. A method of diagnosing a neoplastic tissue of a human, comprising detecting loss of wild-type p53 genes or their expression products in isolated human tissue suspected of being neoplastic, wherein said loss leads to non-functional p53 gene products, loss of expression of p53 mRNA or diminution of expression of p53 mRNA, said loss indicating neoplasia of the tissue, wherein the wild-type p53 gene sequence is shown in Zakut-Houri *et al.,* EMBOJ., 4, 1251-1255, 1985.

2. The method of claim 1 wherein the expression products are mRNA molecules.

3. The method of claim 1 wherein the expression products are protein molecules.

4. The method of claim 1 wherein the loss of wild-type p53 genes is detected by sequencing all or part of the p53 gene using polymerase chain reaction.

5. The method of claim 1 wherein the loss of wild-type p53 genes is detected by identifying a mismatch between molecules (1) a p53 gene or p53 mRNA in said tissue and (2) a nucleic acid probe complementary to the human wild-type p53 gene, when molecules (1) and (2) are hybridized to each other to form a duplex.

6. The method of claim 5 wherein the nucleic acid probe is a RNA probe.

7. The method of claim 5 wherein the nucleic acid probe is a DNA probe.

8. The method of claim 5 wherein the mismatch is identified by enzymatic cleavage.

9. The method of claim 5 wherein the mismatch is identified by chemical cleavage.

10. The method of claim 8 wherein the enzymatic cleavage is performed using RNase A or S 1 nuclease.

11. The method of claim 5 wherein the mismatch is identified by observing a shift in electrophoretic mobility of the duplex relative to the mobility of a duplex formed when molecule (2) is hybridized to a wild-type p53 gene or p53 mRNA.

12. The method of claim 1 wherein the loss of wild-type p53 genes is detected by amplification of p53 gene sequences and hybridization of the amplified p53 sequences to nucleic acid probes which are complementary to mutant p53 alleles.

13. The method of claim 1 wherein the loss of wild-type p53 genes is detected by molecular cloning and sequencing all or part of the p53 gene.

14. The method of claim 3 wherein loss of wild-type p53 protein molecules are detected by the loss of ability to complex with an antigen selected from the group consisting of SV-40 large T antigen and adenovirus E1B antigen.

15. The method of claim 1 wherein the detection of loss of wild-type p53 genes comprises screening for a point mutation.

16. The method of claim 15 wherein the point mutation is a missense mutation.

17. The method of claim 1 wherein the detection of loss of wild-type p53 genes comprises screening for a frameshift mutation.

18. The method of claim 1 wherein the detection of loss of wild-type p53 genes comprises screening for a deletion mutation.

19. The method of claim 1 wherein the detection of loss of wild-type p53 genes comprises screening for a point mutation and screening for a deletion mutation.

20. The method of claim 1 wherein the neoplastic tissue is selected from the group consisting of: lung, breast, brain, colorectal, bladder, prostate, liver and stomach tumours.

21. The method of claim 20 wherein the neoplastic tissue is selected from the group consisting of: lung, breast, and colorectal tumours.

22. The method of claim 21 wherein the neoplastic tissue is a colorectal carcinoma.

23. A method of supplying human wild-type p53 gene function to a human cell which has lost said gene function by virtue of mutation in a p53 gene wherein said mutation leads to non-functional p53 gene products, loss of expression of p53 mRNA or diminution of expression of p53 mRNA, wherein the presence of said mutant p53 gene or expression product indicates the presence of a neoplastic tissue in the human, comprising:
introducing *in vitro* a wild-type p53 gene into the cell such that said gene is expressed in the cell, wherein the wild-type p53 gene sequence is shown in Zakut-Houri *et al.,* EMBOJ., 4, 1251-1255, 1985.

24. The method of claim 23 wherein said wild-type p53 gene is expressed to a level higher than any mutant p53 gene present in the cell.

25. The method of claim 23 wherein the wild-type p53 gene introduced recombines with the endogenous mutant p53 gene present in the cell by a double recombination event to correct the p53 gene mutation.

26. A method of supplying human wild-type p53 gene function to a human cell which has lost said gene function by virtue of a mutation in a p53 gene wherein said mutation leads to non-functional p53 gene products, loss of expression of p53 mRNA, or diminution of expression of p53 mRNA, wherein the presence of said mutant p53 gene or expression product indicates the presence of a neoplastic tissue in the human, comprising:
introducing *in vitro* a portion of a wild-type p53 gene into the cell such that said portion is expressed in the cell, said portion encoding a part of the p53 protein which is required for non-neoplastic growth of said cell, wherein the wild-type p53 gene sequence is shown in Zakut-Houri *et al.,* EMBOJ., 4, 1251-1255, 1985.

27. A method of detecting the presence of a neoplastic tissue in a human, comprising:
isolating from a human a body sample selected from the group consisting of stool, urine and sputum;
detecting in said sample a mutant p53 gene or expression product wherein said mutant p53 gene leads to non-functional p53 gene products, loss of expression of p53 mRNA, or diminution of expression of p53 mRNA, wherein the presence of said mutant p53 gene or expression product indicates the presence of a neoplastic tissue in the human, wherein the wild-type p53 gene sequence is shown in Zakut-Houri *et al.,* EMBOJ., 4, 1251-1255, 1985.

28. A method of detecting genetic predisposition to cancer in a human comprising detecting loss of a wild-type p53 gene in DNA isolated from a human sample selected from the group consisting of blood and fetal tissue wherein said loss leads to non-functional p53 gene products, loss of expression of p53 mRNA, or diminution of expression of p53 mRNA, wherein the wild-type p53 gene sequence is shown in Zakut-Houri *et al.,* EMBOJ., 4, 1251-1255, 1985.

## Patentansprüche

1. Verfahren zur Diagnose eines neoplastischen Gewebes eines Menschen, das den Nachweis des Verlusts von Wildtyp p53-Genen oder deren Expressionsprodukten in isoliertem humanem Gewebe umfaßt, von dem man annimmt, daß es neoplastisch ist, wobei der Verlust zu nicht-funktionalen p53-Genprodukten, zum Verlust der Expression von p53 mRNA oder zur Verminderung der Expression von p53 mRNA führt und der Verlust die Neoplasie des Gewebes anzeigt, wobei die Wildtyp p53 Gensequenz in Zakut-Houri et al., EMBOJ., 4, 1251-1255, 1985, gezeigt wird.

2. Verfahren nach Anspruch 1, bei dem die Expressionsprodukte mRNA-Moleküle sind.

3. Verfahren nach Anspruch 1, bei dem die Expressionsprodukte Proteinmoleküle sind.

4. Verfahren nach Anspruch 1, bei dem man den Verlust von Wildtyp p53-Genen durch Sequenzieren aller oder eines Teils der p53-Gene durch Verwendung von Polymerase-Kettenreaktion nachweist.

5. Verfahren nach Anspruch 1, bei dem man den Verlust von Wildtyp p53-Genen durch Identifizieren eines mismatch zwischen Molekülen nachweist, welche (1) ein p53-Gen oder p53-mRNA in dem Gewebe und (2) eine zum humanen Wildtyp p53-Gen komplementäre Nukleinsäuresonde sind, wenn man die Moleküle (1) und (2) zur Bildung eines Duplex miteinander hybridisiert.

6. Verfahren nach Anspruch 5, bei dem die Nukleinsäuresonde eine RNA-Sonde ist.

7. Verfahren nach Anspruch 4, bei dem die Nukleinsäuresonde eine DNA-Sonde ist.

8. Verfahren nach Anspruch 5, bei dem man das mismatch durch enzymatische Spaltung nachweist.

9. Verfahren nach Anspruch 5, bei dem man das mismatch durch chemische Spaltung identifiziert.

10. Verfahren nach Anspruch 8, bei dem man die enzymatische Spaltung unter Verwendung von RNase A oder S1 Nuklease durchführt.

11. Verfahren nach Anspruch 5, bei dem man das mismatch identifiziert, indem man eine Verschiebung der elektrophoretischen Mobilität des Duplex beobachtet verglichen mit der Mobilität eines Duplex, das sich bildet, wenn man Molekül (2) mit einem Wildtyp p53-Gen oder p53-mRNA hybridisiert.

12. Verfahren nach Anspruch 1, bei dem man den Verlust von Wildtyp p53-Genen durch Amplifikation von p53-Gensequenzen und Hybridisierung der amplifizierten p53-Sequenzen mit gegen Nukleinsäuresonden nachweist, die zu mutanten p53-Allelen komplementär sind.

13. Verfahren nach Anspruch 1, bei dem man den Verlust von Wildtyp p53-Genen durch molekulares Klonieren und Sequenzieren aller oder eines Teils der p53-Gene nachweist.

14. Verfahren nach Anspruch 3, bei dem man den Verlust von Wildtyp p53-Proteinmolekülen anhand des Verlusts der Fähigkeit, mit einem Antigen zu komplexieren, das aus der Gruppe bestehend aus SV-40 large T-Antigen und Adenovirus E1B-Antigen ausgewählt ist, nachweist.

15. Verfahren nach Anspruch 1, bei dem der Nachweis des Verlusts von Wildtyp p53-Genen das Screenen nach einer Punktmutation umfaßt.

16. Verfahren nach Anspruch 15, bei dem die Punktmutation eine missense-Mutation ist.

17. Verfahren nach Anspruch 1, bei dem der Nachweis des Verlusts von Wildtyp p53-Genen das Screenen nach einer frameshift-Mutation umfaßt.

18. Verfahren nach Anspruch 1, bei dem der Nachweis des Verlusts von Wildtyp p53-Genen das Screenen nach einer Deletionsmutation umfaßt.

19. Verfahren nach Anspruch 1, bei dem der Nachweis des Verlusts von Wildtyp p53-Genen das Screenen nach einer Punktmutation und das Screenen nach einer Deletionsmutation umfaßt.

20. Verfahren nach Anspruch 1, bei dem das neoplastische Gewebe aus der Gruppe bestehend aus Lungen-, Brust-, Gehirn-, Colorectal-, Blasen-, Prostata-, Leber- und Magen-Tumoren ausgewählt ist.

21. Verfahren nach Anspruch 20, bei dem das neoplastische Gewebe aus der Gruppe bestehend aus Lungen-, Brust- und Colorectal-Tumoren ausgewählt ist.

22. Verfahren nach Anspruch 21, bei dem das neoplastische Gewebe ein Colorectal-Karzinom ist.

23. Verfahren zum Ausstatten einer menschlichen Zelle mit einer menschlichen Wildtyp p53-Genfunktion, welche diese Genfunktion aufgrund einer Mutation in einem p53-Gen verloren hat und wobei diese Mutation zu nicht-funktionsfähigen p53-Genprodukten, zum Verlust der Expression von p53-mRNA oder zur Verminderung der p53-mRNA-Expression führt und wobei das Vorliegen des mutierten p53-Gens oder -Expressionsprodukts das Vorliegen eines neoplastischen Gewebes in dem Menschen anzeigt, bei dem man in vitro das Wildtyp p53-Gen in die Zelle einbringt, so daß das Gen in der Zelle exprimiert wird, wobei die Wildtyp p53 Gensequenz in Zakut-Houri et al., EMBOJ., 4, 1251-1255, 1985, gezeigt wird.

24. Verfahren nach Anspruch 23, bei dem das Wildtyp p53-Gen zu einem höheren Anteil exprimiert wird als irgendein in der Zelle vorliegendes mutantes p53-Gen.

25. Verfahren nach Anspruch 23, bei dem das eingebrachte Wildtyp p53-Gen mit dem in der Zelle vorliegenden endogenen mutanten p53-Gen durch ein doppeltes Rekombinationsereignis rekombiniert, um die p53-Genmutation zu korrigieren.

26. Verfahren zum Ausstatten einer Zelle mit einer Wildtyp p53-Genfunktion, welche diese Genfunktion aufgrund einer Mutation in einem p53-Gen verloren hat und wobei die Mutation zu nicht-funktionsfähigen p53-Genprodukten, zum Verlust der Expression von p53-mRNA oder zur Verminderung der p53-mRNA-Expression führt und wobei das Vorliegen dieses mutierten p53-Gens oder Expressionsprodukts das Vorliegen eines neoplastischen Gewebes in dem Menschen anzeigt, bei dem man in vitro einen Abschnitt eines Wildtyp p53-Gens in die Zelle hinein-bringt, so daß dieser Abschnitt in der Zelle exprimiert wird, wobei der Abschnitt einen Teil des p53-Proteins kodiert, der für das nicht-neoplastische Wachstum der Zelle erforderlich ist und wobei die Wildtyp p53 Gensequenz in Zakut-Houri et al., EMBOJ., 4, 1251-1255, 1985, gezeigt wird.

27. Verfahren zum Nachweis eines neoplastischen Gewebes in einem Menschen, das das Isolieren einer Körperprobe aus einem Menschen und das Nachweisen eines mutierten p53-Gens oder -Expressionsprodukts in der Probe umfaßt, wobei das mutierte p53-Gen zu nicht-funktionsfähigen p53-Genprodukten, zum Verlust der Expression von p53-mRNA oder zur Verminderung der p53-mRNA-Expression führt, wobei das Vorliegen des mutierten p53-Gens oder -Expressionsprodukts das Vorliegen eines neoplastischen Gewebes in dem Menschen anzeigt und wobei die Wildtyp p53 Gensequenz in Zakut-Houri et al., EMBOJ., 4, 1251-1255, 1985, gezeigt wird.

28. Verfahren zum Nachweis einer genetischen Prädisposition gegenüber Krebs in einem Menschen, das den Nachweis des Verlusts eines Wildtyp p53-Gens in DNA umfaßt, die aus einer humanen Probe isoliert wurde, die aus der Gruppe bestehend aus Blut und fetalem Gewebe ausgewählt ist, wobei der Verlust zu nicht-funktionsfähigen p53-Genprodukten, zum Verlust der Expression von p53-mRNA oder zur Verminderung der p53-mRNA-Expression führt und wobei die Wildtyp p53 Gensequenz in Zakut-Houri et al., EMBOJ., 4, 1251-1255, 1985, gezeigt wird.

## Revendications

1. Procédé permettant de diagnostiquer un tissu néoplasique chez l'homme, qui comporte le fait de détecter la perte de gènes p53 de type sauvage ou des produits de leur expression dans un tissu humain isolé, suspecté d'être néoplasique, ladite perte aboutissant à la formation de produits de gène p53 non-fonctionnels, à la suppression de l'expression d'ARNm de p53 ou à la diminution de l'expression d'ARNm de p53, et ladite perte indiquant le caractère néoplasique du tissu, étant entendu que la séquence du gène p53 de type sauvage est celle indiquée dans l'article de Zakut-Houri et coll., EMBOJ 4, 1251-1255 (1985).

2. Procédé conforme à la revendication 1, dans lequel les produits d'expression sont des molécules d'ARNm.

3. Procédé conforme à la revendication 1, dans lequel les produits d'expression sont des molécules de protéines.

4. Procédé conforme à la revendication 1, dans lequel on détecte la perte de gènes p53 de type sauvage en séquençant tout ou partie du gène p53 à l'aide d'une réaction en chaîne avec polymérase (PCR).

5. Procédé conforme à la revendication 1, dans lequel on détecte la perte de gènes p53 de type sauvage en identifiant un défaut d'appariement entre les molécules que sont (1) un gène p53 ou un ARNm de p53 provenant dudit tissu et (2) une sonde d'acide nucléique complémentaire du gène p53 humain de type sauvage, lorsqu'on hybride l'une avec l'autre les molécules (1) et (2) pour en former un duplex.

6. Procédé conforme à la revendication 5, dans lequel la sonde d'acide nucléique est une sonde d'ARN.

7. Procédé conforme à la revendication 5, dans lequel la sonde d'acide nucléique est une sonde d'ADN.

8. Procédé conforme à la revendication 5, dans lequel le défaut d'appariement est identifié par coupure enzymatique.

9. Procédé conforme à la revendication 5, dans lequel le défaut d'appariement est identifié par coupure chimique.

10. Procédé conforme à la revendication 8, dans lequel la coupure enzymatique est effectuée à l'aide d'ARNase ou de nucléase S1.

11. Procédé conforme à la revendication 5, dans lequel on identifie le défaut d'appariement en observant la différence de mobilité du duplex en électrophorèse par rapport à la mobilité d'un duplex formé par hybridation d'une molécule (2) avec un gène p53 de type sauvage ou un ARNm de p53 de type sauvage.

12. Procédé conforme à la revendication 1, dans lequel on détecte la perte de gènes p53 de type sauvage par amplification de séquences du gène p53 et hybridation de ces séquences de gène p53 amplifiées avec des sondes d'acide nucléique qui sont complémentaires d'allèles mutants du gène p53.

13. Procédé conforme à la revendication 1, dans lequel on détecte la perte de gènes p53 de type sauvage par clonage moléculaire et séquençage de tout ou partie du gène p53.

14. Procédé conforme à la revendication 3, dans lequel on détecte la perte des molécules de protéines p53 de type sauvage par la perte de leur capacité à former des complexes avec un antigène choisi dans l'ensemble constitué par l'antigène grand T de SV-40 et l'antigène E1B d'adénovirus.

15. Procédé conforme à la revendication 1, dans lequel la détection de la perte de gènes p53 de type sauvage comporte la recherche par criblage d'une mutation ponctuelle.

16. Procédé conforme à la revendication 15, dans lequel la mutation ponctuelle est une mutation faux-sens.

17. Procédé conforme à la revendication 1, dans lequel la détection de la perte de gènes p53 de type sauvage comporte la recherche par criblage d'une mutation par décalage du cadre de lecture.

18. Procédé conforme à la revendication 1, dans lequel la détection de la perte de gènes p53 de type sauvage comporte la recherche par criblage d'une mutation par délétion.

19. Procédé conforme à la revendication 1, dans lequel la détection de la perte de gènes p53 de type sauvage comporte la recherche par criblage d'une mutation ponctuelle et la recherche par criblage d'une mutation par délétion.

20. Procédé conforme à la revendication 1, dans lequel le tissu néoplasique est choisi dans l'ensemble que constituent des tumeurs du poumon, du sein, du cerveau, du côlon-rectum, de la vessie, de la prostate, du foie ou de l'estomac.

21. Procédé conforme à la revendication 20, dans lequel le tissu néoplasique est choisi dans l'ensemble que constituent des tumeurs du poumon, du sein ou du côlon-rectum.

22. Procédé conforme à la revendication 21, dans lequel le tissu néoplasique provient d'un cancer du côlon-rectum.

23. Procédé permettant de restaurer la fonction du gène p53 humain de type sauvage chez une cellule humaine qui a perdu la fonction de ce gène en raison d'une mutation intervenue dans ce gène p53, laquelle mutation aboutit à la formation de produits de gène p53 non-fonctionnels, à la suppression de l'expression d'ARNm de p53 ou à la diminution de l'expression d'ARNm de p53, la présence dudit gène p53 muté ou d'un produit de son expression étant l'indice de la présence d'un tissu néoplasique chez l'homme, lequel procédé comporte :
le fait d'introduire *in vitro* un gène p53 de type sauvage dans la cellule de telle façon que ce gène puisse s'exprimer au sein de cette cellule, étant entendu que la séquence du gène p53 de type sauvage est celle indiquée dans l'article de Zakut-Houri et coll., EMBOJ 4, 1251-1255 (1985).

24. Procédé conforme à la revendication 23, dans lequel ledit gène p53 de type sauvage s'exprime à un niveau élevé que n'importe quel gène p53 muté présent dans la cellule.

25. Procédé conforme à la revendication 23, dans lequel le gène p53 de type sauvage introduit se recombine par double recombinaison avec le gène p53 muté endogène, déjà présent dans la cellule, de manière à corriger la mutation du gène p53.

26. Procédé permettant de restaurer la fonction du gène p53 humain de type sauvage chez une cellule humaine qui a perdu la fonction de ce gène en raison d'une mutation intervenue dans ce gène p53, laquelle mutation aboutit à la formation de produits de gène p53 non-fonctionnels, à la suppression de l'expression d'ARNm de p53 ou à la diminution de l'expression d'ARNm de p53, la présence dudit gène p53 muté ou d'un produit de son expression étant l'indice de la présence d'un tissu néoplasique chez l'homme, lequel procédé comporte :
le fait d'introduire *in vitro* une fraction du gène p53 de type sauvage dans la cellule de telle façon que cette fraction puisse s'exprimer au sein de cette cellule, ladite fraction codant une partie de la protéine p53 qui est nécessaire pour que ladite cellule prolifère de manière non-néoplasique. étant entendu que la séquence du gène p53 de type sauvage est celle indiquée dans l'article de Zakut-Houri et coll., EMBOJ 4, 1251-1255 (1985).

27. Procédé permettant de détecter la présence d'un tissu néoplasique chez l'homme, qui comporte :
le fait d'isoler un échantillon corporel chez un être humain, lequel échantillon corporel est choisi dans l'ensemble formé par les selles, l'urine et les expectorations,
et le fait de détecter dans cet échantillon un gène p53 muté ou le produit de l'expression de celui-ci, ledit gène p53 muté provoquant la formation de produits de gène p53 non-fonctionnels, la suppression de l'expression d'ARNm de p53 ou la diminution de l'expression d'ARNm de p53, et la présence dudit gène p53 uté ou produit de son expression étant l'indice de la présence tissu néoplasique chez l'homme, étant entendu que la séquence du gène p53 de type sauvage est celle indiquée dans l'article de Zakut-Houri et coll., EMBOJ 4, 1251-1255 (1985).

28. Procédé permettant de détecter chez l'homme une prédisposition génétique au cancer, qui comporte le fait de détecter la perte d'un gène p53 de type sauvage dans de l'ADN isolé à partir d'un échantillon humain choisi parmi du sang et du tissu foetal, ladite perte aboutissant à la formation de produits de gène p53 non-fonctionnels, à la suppression de l'expression d'ARNm de p53 ou à la diminution de l'expression d'ARNm de p53, étant entendu que la séquence du gène p53 de type sauvage est celle indiquée dans l'article de Zakut-Houri et coll., EMBOJ 4, 1251-1255 (1985).
